(19) Europäisches Patentamt
European Patent Office
Office européen des brevets

(11) Veröffentlichungsnummer : **0 065 107**
**B1**

(12) **EUROPÄISCHE PATENTSCHRIFT**

(45) Veröffentlichungstag der Patentschrift : **18.09.85**

(21) Anmeldenummer : **82103202.6**

(22) Anmeldetag : **16.04.82**

(51) Int. Cl.⁴ : **C 07 D249/08**, C 07 D233/61, A 01 N 43/64, A 01 N 43/50, C 07 D405/12

(54) **Azolylpropyl-oximino-Derivate, Verfahren zu ihrer Herstellung sowie ihre Verwendung als Fungizide und Pflanzenwachstumsregulatoren.**

(30) Priorität : **28.04.81 DE 3116888**

(43) Veröffentlichungstag der Anmeldung : **24.11.82 Patentblatt 82/47**

(45) Bekanntmachung des Hinweises auf die Patenterteilung : **18.09.85 Patentblatt 85/38**

(84) Benannte Vertragsstaaten : **AT BE CH DE FR GB IT LI NL**

(56) Entgegenhaltungen :
**EP-A- 0 005 249**
**EP-A- 0 009 740**
**DE-A- 2 657 578**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Elbe, Hans-Ludwig, Dr.**
**Dasnöckel 59**
**D-5600 Wuppertal 11 (DE)**
Erfinder : **Büchel, Karl Heinz, Dr. Prof.**
**Dabringhausenerstrasse 42**
**D-5093 Burscheid (DE)**
Erfinder : **Frohberger, Paul-Ernst, Dr.**
**Willi-Baumeister-Strasse 5**
**D-5090 Leverkusen 1 (DE)**
Erfinder : **Brandes, Wilhelm, Dr.**
**Eichendorffstrasse 3**
**D-5653 Leichlingen 1 (DE)**
Erfinder : **Lürssen, Klaus, Dr.**
**August-Kierspel-Strasse 89**
**D-5060 Bergisch-Galdbach 2 (DE)**

**EP 0 065 107 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Azolylpropyl-oximino-Derivate, mehrere Verfahren zu ihrer Herstellung sowie ihre Verwendung als Fungizide und Pflanzenwachstumsregulatoren.

Es ist bereits bekannt geworden, daß substituierte 2-Azolyl-1-benzyloximino-1-phenyl-ethane gute fungizide Eigenschaften aufweisen (vergleiche DE-OS 26 57 578, DE-OS 27 23 924 und DE-OS 28 16 817). Die Wirkung dieser Verbindungen ist jedoch, insbesondere bei niedrigen Aufwandmengen und -konzentrationen, nicht immer voll befriedigend.

Es wurden neue Azolylpropyl-oximino-Derivate der allgemeinen Formel

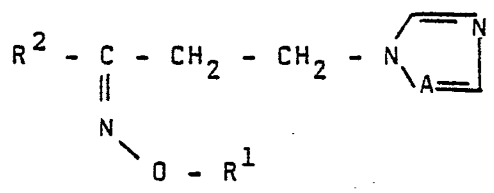

(I)

in welcher

A für Stickstoff oder die CH-Gruppe steht,

$R^1$ für Alkyl mit 1 bis 8 Kohlenstoffatomen, Alkenyl und Alkinyl mit jeweils 2 bis 6 Kohlenstoffatomen steht, sodann für Cyclopentyl, Cyclohexyl, Cyclohexylmethyl und Cyclohexenyl steht, sowie für gegebenenfalls einfach bis dreifach, gleichartig oder verschieden, durch Fluor, Chlor, Brom, Cyano, Nitro, Methyl, Ethyl, Methoxy, Methylendioxo, Phenyl, Phenoxy und/oder Trifluormethyl substituiertes Benzyl oder Phenyl steht, und

$R^2$ für substituiertes Phenyl steht, welches einfach oder zweifach, gleichartig oder verschieden durch Fluor, Chlor, Brom und Methyl, sowie durch Phenyl oder Phenoxy substituiert ist, wobei die beiden letztgenannten Zweitsubstituenten selbst noch gegebenenfalls durch Chlor, Brom oder Nitro substituiert sein können,

sowie deren physiologisch verträglichen Säureadditions-Salze und Metallsalz-Komplexe gefunden.

Die Verbindungen der Formel (I) können in der syn- oder anti- Form vorliegen ; vorwiegend fallen sie als Gemische beider Formen an.

Weiterhin wurde gefunden, daß man die Azolylpropyl-oximino-Derivate der Formel (I) erhält, wenn man

a) Azolyl-ketone der Formel

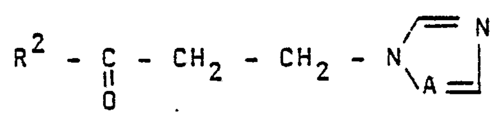

(II)

in welcher

A und $R^2$ die oben angegebene Bedeutung haben,

mit substituierten Hydroxylaminen der Formel

$$H_2N—O—R^1 \qquad (III)$$

in welcher

$R^1$ die oben angegebene Bedeutung hat,

in Gegenwart eines Verdünnungsmittels umsetzt, oder

b) Azolyl-oxime der Formel

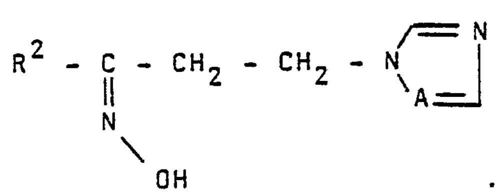

(IV)

in welcher

A und $R^2$ die oben angegebene Bedeutung haben,

mit Halogeniden der Formel

Hal—$R^1$ (V)

in welcher

$R^1$ die oben angegebene Bedeutung hat und

Hal für Chlor oder Brom steht,

gegebenenfalls in Gegenwart einer starken Base und in Gegenwart eines Verdunnungsmittels umsetzt, oder

c) Halogenpropyl-oximino-Derivate der Formel

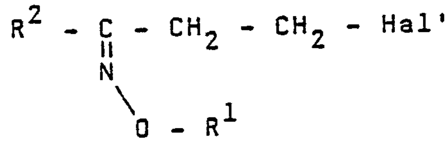

(VI)

in welcher

$R^1$ und $R^2$ die oben angegebene Bedeutung haben und

Hal' für Chlor oder Brom steht,

mit 1,2,4-Triazol oder Imidazol in Gegenwart eines Säurebindemittels und in Gegenwart eines Verdünnungsmittels umsetzt.

An die so erhaltenen Verbindungen der Formel (I) kann gegebenenfalls eine Säure oder ein Metallsalz addiert werden.

Schließlich wurde gefunden, daß die neuen Azolylpropyl-oximino-Derivate der Formel (I) sowie deren Säureadditionssalze und Metallsalz-komplexe starke fungizide und pflanzenwachstumsregulierende Eigenschaften aufweisen.

Überraschenderweise zeigen die erfindungsgemäßen Verbindungen der Formel (I) eine bessere fungizide Wirksamkeit als die aus dem Stand der Technik bekannten substituierten 2-Azolyl-1-benzyloximino-1-phenyl-ethane, welche chemisch und wirkungsmäßig naheliegende Verbindungen sind. Zusätzlich zeigen die erfindungsgemäßen Verbindungen der Formel (I) überraschend gute pflanzenwachstumsregulierende Wirkung. Die erfindungsgemäßen Wirkstoffe stellen somit eine Bereicherung der Technik dar.

Die erfindungsgemäßen Azolylpropyl-oximino-Derivate sind durch die Formel (I) definiert.

Im einzelnen seien außer den bei den Herstellungsbeispielen genannten Verbindungen die folgenden Verbindungen der Formel (I) genannt

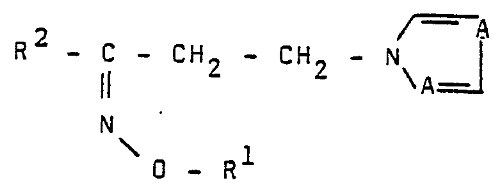

(A= N, CH)

| $R^2$ | $R^1$ |
|---|---|
| 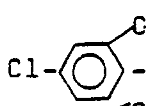 | 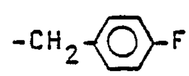 |
| 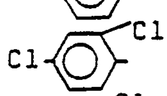 | 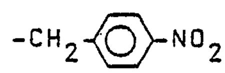 |
| 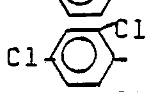 | 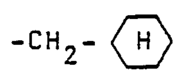 |
| 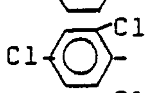 | $-CH_2-CH=CH_2$ |
| 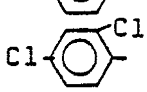 | $-CH_2-C\equiv CH$ |

(Fortsetzung)

| $R^2$ | $R^1$ |
|---|---|
| 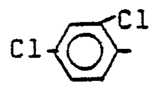 | 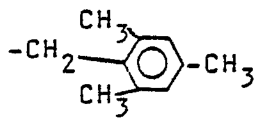 |
| 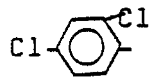 | 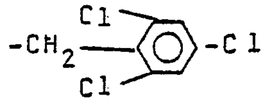 |
| 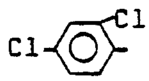 | 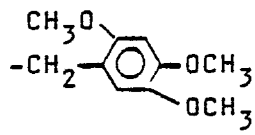 |
| 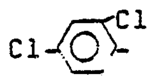 | 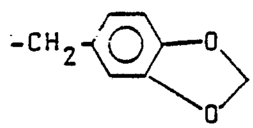 |
| 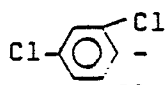 | 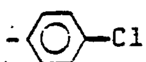 |
| 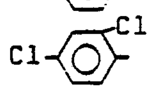 | 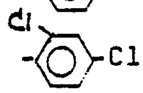 |
| 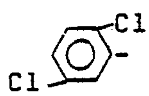 | 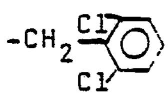 |
| 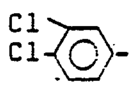 | 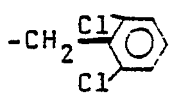 |
| 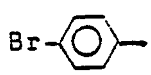 | 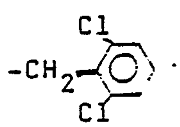 |
| 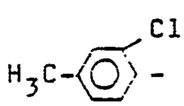 | 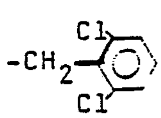 |
| 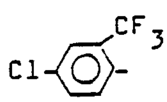 | 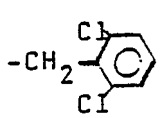 |
| 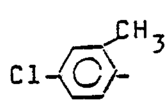 | 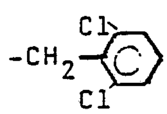 |

(Fortsetzung)

| $R^2$ | $R^1$ |
|---|---|
| 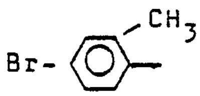 | 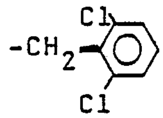 |
| 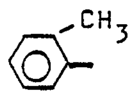 | 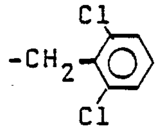 |

Verwendet man beispielsweise (4,4'-Chlorbiphenyl-yl)-(2-imidazol-1-yl-ethyl)-keton und 0-(2,6-Dichlorbenzyl)-hydroxylamin-hydrochlorid als Ausgangstoffe, so kann der Reaktionsablauf durch das folgende Formelschema wiedergegeben werden (Verfahren a) :

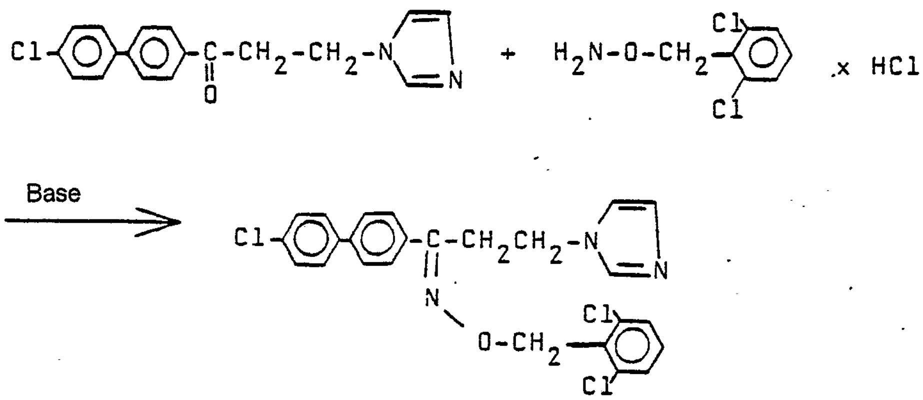


Verwendet man beispielsweise 1-(2,4-Dichlorphenyl)-1-oximino-3-(1,2,4-triazol-1-yl)-propan und 2,4-Dichlorbenzylchlorid als Ausgangsstoffe, so kann der Reaktionsablauf durch das folgende Formelschema wiedergegeben werden (Verfahren b) :

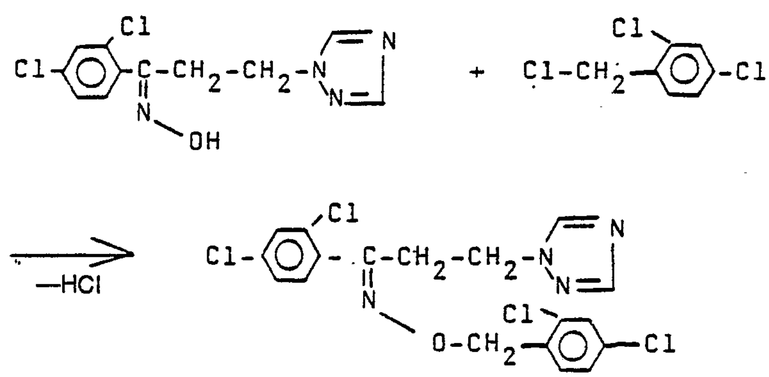


Verwendet man beispielsweise 3-Chlor-1-(2,4-dichlorphenyl)-1-methyloximino-propan und 1,2,4-Triazol als Ausgangsstoffe, so kann der Reaktionsablauf durch das folgende Formelschema wiedergegeben werden (Verfahren c) :

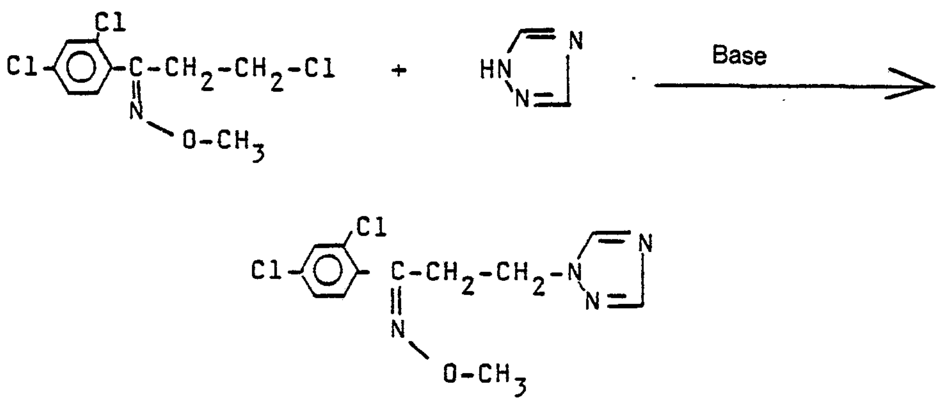


Die bei der Durchführung des erfindungsgemäßen Verfahrens (a) als Ausgangsstoffe benötigten Azolylketone sind durch die Formel (II) allgemein definiert. In dieser Formel haben A und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Azolyl-ketone der Formel (II) sind teilweise beschrieben (vergleiche DE-OS 26 33 492 bzw. US-Patenschrift 4 045 568). Neu sind jedoch solche Derivate, in denen A für Stickstoff steht. Sie können nach den dort angegebenen Verfahren erhalten werden, indem man z. B. Vinylketone der Formel

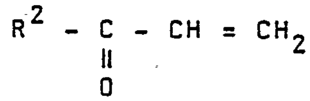
(VII)

in welcher

$R^2$ die oben angegebene Bedeutung hat,

in üblicher Weise mit 1,2,4-Triazol oder Imidazol in Gegenwart eines inerten, organischen Lösungsmittels, wie z. B. Dimethylformamid, bei Temperaturen zwischen 0 und 100 °C umsetzt, oder Halogenmethylketone der Formel

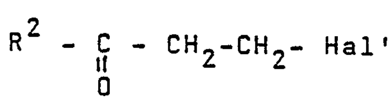
(VIII)

in welcher

$R^2$ und Hal' die oben angegebene Bedeutung haben,

in üblicher Weise mit 1,2,4-Triazol oder Imidazol in Gegenwart eines Säurebindemittels, wie z. B. Kaliumcarbonat, und in Gegenwart eines inerten organischen Lösungsmittels, wie z. B. Dimethylformamid, bei Temperaturen zwischen 40 und 120 °C umsetzt.

Die außerdem für das erfindungsgemäße Verfahren (a) als Ausgangsstoffe benötigten substituierten Hydroxylamine sind durch die Formel (II) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden.

Die substituierten Hydroxylamine der Formel (III) sind allgemeinen bekannte Verbindungen der organischen Chemie.

Die bei der Durchführung des erfindungsgemäßen Verfahrens (b) als Ausgangsstoffe benötigten Azolyl-oxime sind durch die Formel (IV) allgemein definiert. In dieser Formel haben A und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Azolyl-oxime der Formel (IV) sind noch nicht bekannt. Sie können jedoch in allgemein bekannter Art und Weise erhalten werden, indem man Azolylketone der Formel (II) mit Hydroxylamin in Gegenwart eines Lösungsmittels, vorzugsweise eines Alkohols, bei 50 bis 100 °C umsetzt, wobei das Hydroxylamin vorzugsweise als Hydrochlorid in Gengenwart eines Säurebindemittels eingesetzt wird.

Die außerdem für das erfindungsgemäße Verfahren (b) als Ausgangsstoffe benötigten Halogenide sind durch die Formel (V) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Halogenide der Formel (V) sind allgemein bekannte Verbindungen der organischen Chemie.

Die bei der Durchführung des erfindungsgemäßen Verfahrens (c) als Ausgangsstoffe benötigten Halogenpropyl-oximino-Derivate sind durch die Formel (VI) allgemein definiert. In dieser Formel stehen $R^1$ und $R^2$ vorzugsweise für diejenigen Reste, die bereits im Zusammenhang mit der Beschreibung der

erfindungsgemäßen Stoffe der Formel (I) vorzugsweise für diese Substituenten genannt wurden.

Die Halogenpropyl-oximino-Derivate der Formel (VI) sind noch nicht bekannt. Sie können jedoch in allgemein bekannter Art und Weise erhalten werden, indem man Halogenethyl-ketone der Formel (VIII) mit substituierten Hydroxylaminen der Formel (III) in Gegenwart eines Lösungsmittels, vorzugsweise eines Alkohols, bei 50 bis 100 °C umsetzt, wobei die substituierten Hydroxylamine vorzugsweise als Hydrochloride in Gegenwart eines Säurebinders eingesetzt werden.

Als Verdünnungsmittel kommen für das erfindungsgemäße Verfahren (a) vorzugsweise Alkohole und Wasser, bzw. Gemische beider infrage.

Die Reaktionstemperaturen können beim Verfahren (a) in einem größeren Bereich variiert werden. Im allgemeinen arbeitet man zwischen 20 und 120 °C, vorzugsweise zwischen 50 und 100 °C.

Bei der Durchführung des erfindungsgemäßen Verfahrens (a) setzt man auf 1 mol Azolylketon der Formel (II) vorzugsweise 1 bis 1,3 Mol Hydroxylamin der Formel (III) ein. Die Isolierung der Verbindungen der Formel (I) erfolgt nach üblichen Methoden.

Nach einer bevorzugten Ausführungsform des Verfahrens (a) werden die Hydroxylamine der Formel (III) in Form ihrer Salze, insbesondere als Hydrochloride, gegebenenfalls in Gegenwart eines Säurebindemittels, wie beispielsweise Natriumacetat, eingesetzt (vergleiche auch die Herstellungsbeispiele).

Für die erfindungsgemäße Umsetzung gemäß Verfahren (b) kommen als Verdünnungsmittel inerte organische Lösungsmittel infrage. Hierzu gehören vorzugsweise Ether, wie Tetrahydrofuran and Dioxan ; aromatische Kohlenwasserstoffe, wie Toluol und Benzol ; in einzelnen Fällen auch chlorierte Kohlenwasserstoffe, wie Chloroform, Methylenchlorid oder Tetrachlorkohlenstoff ; sowie Hexamethylphosphorsäure-triamid, Säureamide, wie Dimethylformamid und Sulfoxide, wie Dimethylsulfoxid.

Die erfindungsgemäße Umsetzung gemäß Verfahren (b) wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quaternäre Ammoniumhydroxide und Phosphonium-hydroxide, wie beispielsweise Tetramethylammoniumhydroxid, Benzyltrimethyl-ammoniumhydroxid oder Dibenzyldimethyl-ammoniumhydroxid und Tetraphenylphosphoniumhydroxid oder Methyl-triphenylphosphoniumhydroxid.

Die Reaktionstemperaturen können beim Verfahren (b) in einem größeren Bereich variiert werden. Im allgemeinen arbeitet man zwischen 20 und 150 °C, vorzugsweise bei Raumtemperatur. In einzelnen Fällen ist es vorteilhaft bei der Siedetemperatur des Lösungsmittels, beispielsweise zwischen 60 und 100 °C, zu arbeiten.

Bei der Durchführung des erfindungsgemäßen Verfahrens (b) setzt man auf 1 Mol Oxim der Formel (IV) vorzugsweise 1 bis 3 Mol Halogenid der Formel (V) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform des Verfahrens (b) wird die erfindungsgemäße Umsetzung in einem Zweiphasensystem, wie beispielsweise wässrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Ethylate entstehen und mit den in der organischen Phase befindlichen Halogeniden umgesetzt werden.

Für die erfindungsgemäße Umsetzung gemäß Verfahren (c) kommen als Verdünnungsmittel vorzugsweise inerte organische Lösungsmittel infrage. Hierzu gehören Nitrile, wie Acetonitril ; Alkohole, wie Ethanol ; Ether, wie Tetrahydrofuran oder Dioxan ; aromatische Kohlenwasserstoffe, wie Toluol und Benzol ; Formamide, wie Dimethylformamid ; sowie halogenierte Kohlenwasserstoffe, wie Methylenchlorid, Tetrachlorkohlenstoff oder Chloroform.

Die erfindungsgemäße Umsetzung gemäß Verfahren (c) wird in Gegenwart eines Säurebindemittels vorgenommen. Man kann alle üblicherweise verwendbaren anorganischen oder organischen Säurebinder zugeben, wie Alkalicarbonate, z. B. Natriumcarbonat und Kaliumcarbonat ; oder wie niedere tertiäre Alkylamine, Cycloalkylamine oder Aralkylamine, z. B. Triethylamin, N,N-Dimethylcyclohexylamin und N,N-Dimethylbenzylamin. Ebenfalls möglich ist ein entsprechender Überschuß an 1,2,4-Triazol bzw. Imidazol.

Die Reaktionstemperaturen können beim Verfahren (c) in einem größeren Bereich variiert werden. Im allgemeinen arbeitet man zwischen 0 und 150 °C, vorzugsweise zwischen 60 und 120 °C.

Zur Durchführung des erfindungsgemäßen Verfahrens (c) setzt man auf 1 Mol der Verbindungen der Formel (VI) vorzugsweise 1 bis 2 Mol 1,2,4-Triazol bzw. Imidazol und 1 bis 2 Mol Säurebinder ein. Die Isolierung der Verbindungen der Formel (I) erfolgt nach üblichen Methoden.

Gemäß einer besonderen Ausführungsform der Verfahrens (c) kann auch so vorgegangen werden, daß man zunächst die Zwischenprodukte der Formel (VI) herstellt und ohne deren Isolierung und ohne Lösungsmittelwechsel die weitere Umsetzung durchgeführt und die Endprodukte der Formel (I) im Rahmen eines « Eintopfverfahrens » in einem Arbeitsgang erhält.

Zur Herstellung von physiologisch verträglichen Säureadditionssalzen der Verbindungen der Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner

Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salizylsäure, Sorbinsäure, Milchsäure, sowie Sulfonsäuren, wie z. B. p-Toluolsulfonsäure und 1,5-Naphthalindisulfonsäure. Die Säureadditionssalze der Verbindungen der Formel (I) können in einfacher Weise nach üblichen Salzbildungsmethoden, z. B. durch Lösen einer Verbindung der Formel (I) in einem geeigneten organischen Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Mettalsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure. Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Getreidekrankheiten, wie z. B. gegen den Erreger des Gerstenmehltaus (Erysiphe graminis), von Reiskrankheiten, wie z. B. Pyricularia oryzae, von Venturia-Arten, wie z. B. gegen den Erreger des Apfelschorfs (Venturia inaequalis), sowie zur bakteriziden Bekämpfung eingesetzt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pfanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Sträßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt, werden, sodaß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind. z. B. Aerosol-Treibgas, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsaüre, Aluminiumoxid und Silikate. Als feste

Trägerstoffe für Granulate kommen in Frage : z. B. gebrochene und fraktionierte natürliche Gesteine wie Calcit, Marmor, Bims, Sepiolith, Dolimit sowie synthetische Granulate aus anorganischen und organischen Mehlen sowie Granulate aus organischem Material wie Sägmehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol- Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größen Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkungskonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

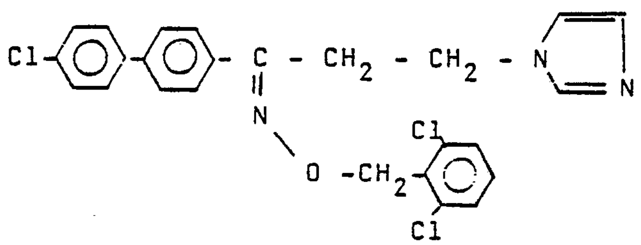

(Verfahren a)

12,4 g (0,04 Mol) (4,4'-Chlorbiphenylyl)-(2-imidazol-1-yl-ethyl)-keton werden mit 13,7 g (0,06 Mol) O-(2,6-Dichlorbenzyl)-hydroxylamin-hydrochlorid und 3,9 g (0,048 Mol) Natriumacetat in 40 ml Wasser 1 Stunde bei 60 °C gerührt. Man läßt das Reaktionsgemisch abkühlen und extrahiert mit Essigsäureethylester. Die organische Phase wird mit Wasser gewaschen, über Natriumsulfat getrocknet und eingeengt. Der Rückstand wird säulenchromatographisch gereinigt. Man erhält 3,9 g (70 % der Theorie) 1-(4,4'-Chlorbiphenylyl)-1-(2,6-dichlorbenzyl-oximino)-3-(imidazol-1-yl)-propan vom Schmelzpunkt 110-111 °C.

### Herstellung des Ausgangsproduktes

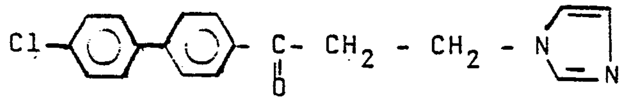

120 g (0,43 Mol) (4,4'-Chlorbiphenylyl)-(2-chlor-ethyl)-keton in 500 ml Aceton werden bei Raumtemperatur unter Rühren zu 32,4 g (0,47 Mol) Imidazol und 178 g (1,29 Mol) Kaliumcarbonat in 500 ml Aceton getropft, wobei die Temperatur auf ca. 35 °C ansteigt. Man läßt 8 Stunden unter Rückfluß rühren und danach abkühlen. Das Reaktionsgemisch wird filtriert. Das Filtrat wird eingeengt, der Rückstand in Methylenchlorid aufgenommen, mit Wasser gewaschen, über Natriumsulfat getrocknet und erneut eingeengt. Der Rückstand wird aus Toluol umkristallisiert. Man erhält 60 g (45 % der Theorie) (4,4'-Chlorbiphenylyl)-(2-imidazol-1-yl-ethyl)-keton vom Schmelzpunkt 132-134 °C.

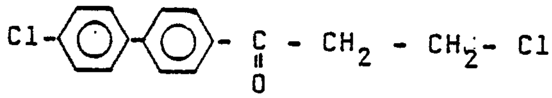

117 g (0,92 Mol) 3-Chlorpropionylchlorid werden bei Raumtemperatur und unter Kühlung zu 170 g (0,9 Mol) 4-Chlorbiphenyl und 144 g (1,08 Mol) wasserfreiem Aluminium-chlorid in 1 000 ml Ethylenchlorid getropft. Man läßt eine Stunde bei 25 °C nachrühren und gibt das Reaktionsgemisch anschließend in 3 l Eiswasser. Die organische Phase wird abgetrennt, über Natriumsulfat getrocknet und eingeengt. Der Rückstand wird aus Diisopropylether umkristallisiert. Man erhält 242 g (96,3 % der Theorie) (4-4'-Chlorbiphenylyl)-(2-chlorethyl)-keton vom Schmelzpunkt 90-94 °C.

In entsprechender Weise und gemäß den angegebenen Verfahren werden die folgenden Verbindungen der allgemeinen Formel

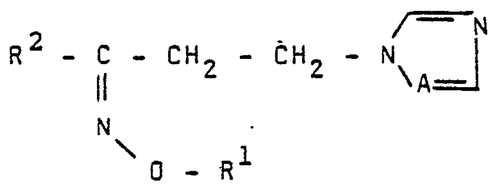 (I)

erhalten :

| Bsp. Nr. | $R^1$ | $R^2$ | A | Schmelzpunkt (°C) bzw. Brechungsindex $n_D^{20}$ |
|---|---|---|---|---|
| 2 | 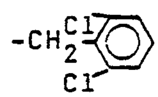 | 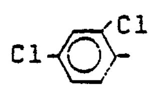 | N | 1,5937 |
| 3 | 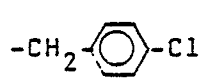 | 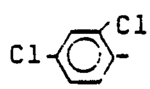 | N | 1,5902 |
| 4 | 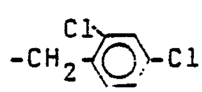 | 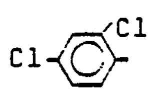 | N | 1,5912 |
| 5 | $-C_4H_9$-n | 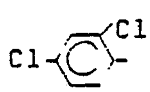 | N | 1,5491 |
| 6 | 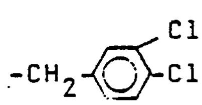 | 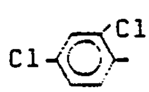 | N | 1,5937 |
| 7 | 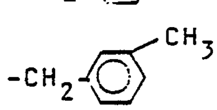 | 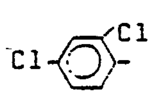 | N | 1,5814 |
| 8 | $-CH_3$ | 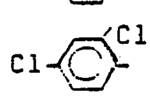 | N | 1,5665 |

(Fortsetzung)

| Bsp. Nr. | $R^1$ | $R^2$ | A | Schmelzpunkt (°C) bzw. Brechungsindex $n_D^{20}$ |
|---|---|---|---|---|
| 9 | $-C_4H_9-n$ | 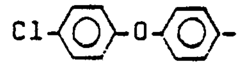 | N | 1,5760 |
| 10 | 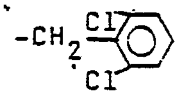 | 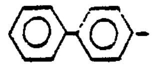 | N | zähes Oel |
| 11 | 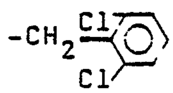 | 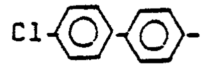 | N | 138-40 |
| 12 | 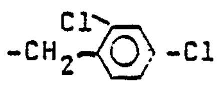 | 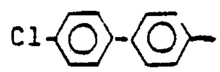 | N | 130-31 |
| 13 | 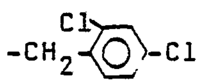 | 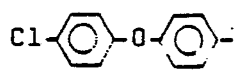 | N | 1,6100 |
| 14 | 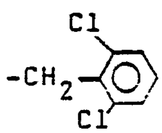 | 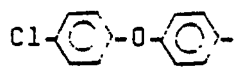 | N | 1,6134 |
| 15 | $-C_4H_9-n$ | 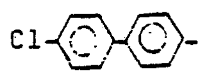 | N | 72-73 (Form-A)* |
| 16 | $-C_4H_9-n$ | 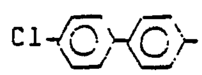 | N | zähes Oel* (Form B)* |
| 17 | 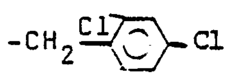 | 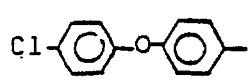 | CH | 1,6165 |
| 18 | 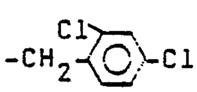 | 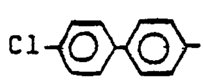 | CH | 154-156 |
| 19 | 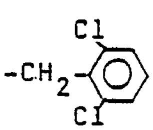 | 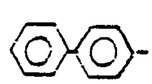 | CH | 190 |

(Fortsetzung)

| Bsp. Nr. | $R^1$ | $R^2$ | A | Schmelzpunkt (°C) bzw. Brechungsindex $n_D^{20}$ |
|---|---|---|---|---|
| 20 | $-C_7H_{15}-n$ | 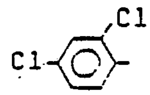 | CH | 1,5390 |
| 21 | 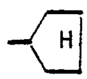 | 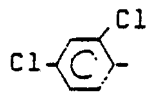 | CH | 1,5684 |
| 22 | $-C_4H_9-n$ | 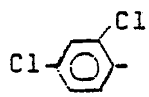 | CH | 1,5601 |
| 23 | $-C_4H_9-n$ | 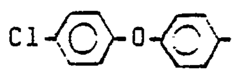 | CH | 1,5756 |
| 24 | $-C_4H_9-n$ | 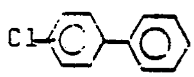 | CH | 1,6088 |
| 25 | $-C_4H_9-n$ | 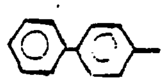 | CH | 1,5923 |
| 26 | $-CH(CH_3)C_2H_5$ | 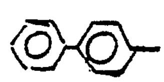 | CH | 1,5880 |
| 27 | 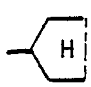 | 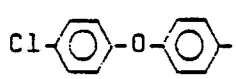 | CH | 1,5972 |
| 28 | 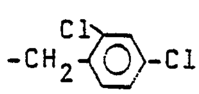 | 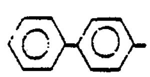 | CH | 1,6244 |
| 29 | $-C_7H_{15}-n$ | 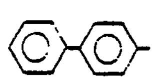 | CH | 1,5710 |

(Fortsetzung)

| Bsp. Nr. | $R^1$ | $R^2$ | A | Schmelzpunkt (°C) bzw. Brechungs-index $n_D^{20}$ |
|---|---|---|---|---|
| 30 | $-CH_3$ | 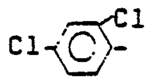 | CH | 1,5842 |
| 31 | 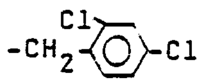 | 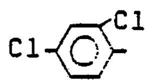 | CH | 1,5992 |
| 32 | 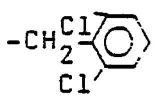 | 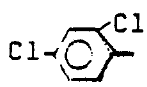 | CH | 1,5992 |
| 33 | 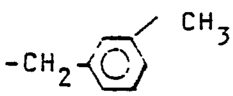 | 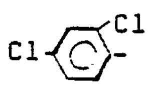 | CH | 1,5878 |
| 34 | 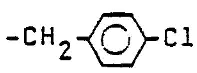 | 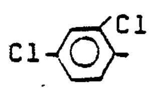 | CH | 1,5878 |
| 35 | 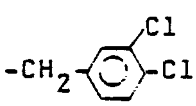 | 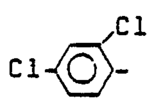 | CH | 1,5920 |
| 36 | 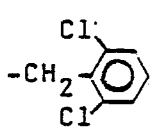 | 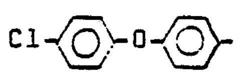 | CH | 1,6200 |
| 37 | 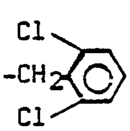 | 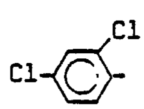 | N | 85-90 (x $CuCl_2$) |

*Formen A und B : die beiden möglichen geometrischen Isomeren

Anwendungsbeispiele

In den nachfolgenden fungizide Anwendungsbeispielen werden die nachstehend angegebenen Verbindungen als Vergleichssubstanzen eingesetzt.

(A) 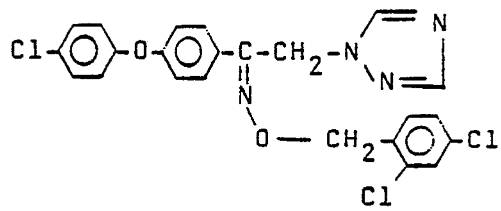

(B) 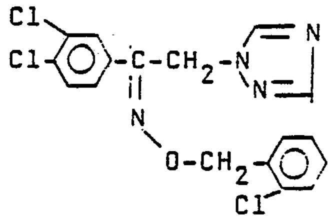

(C) 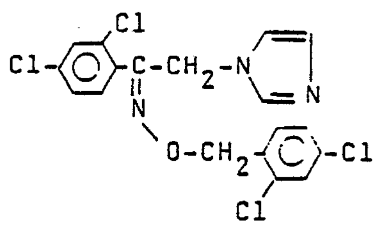

× $HNO_3$ syn-Form

(D) 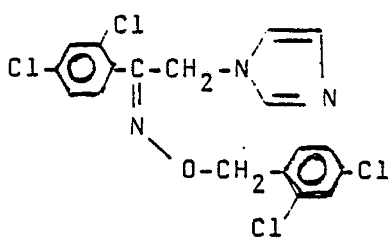

× $HNO_3$ anti-Form

Beispiel A

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 2, 3, 4, 5, 6, 7, 8, 9, 10, 11, 15, 16, 32, 30, 22, 31, 33, 34, 35, 24, 25, 26, 21, 20, und 18.

Beispiel B

Venturia-Test (Apfel)/protektiv

Lösungsmittel : 4,7 Gewichtsteile Aceton

Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer waßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 2, 3, 4, 32, 31 und 30.

Beispiel C

Wuchshemmung bei Zuckerrüben

Lösungsmittel : 30 Gewichtsteile Dimethylformamid

Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0 % Wuchshemmung ein Wachstum entsprechend dem der Kontrollpflanzen. 100 % Wuchshemmung bedeutet Stillstand des Wachstums.

Die erfindungsgemäßen Wirkstoffe 9, 14, 15, 17 zeigen in diesem Test eine gute Wuchsbeeinflussung im Vergleich zur Kontrolle.

**Patentansprüche**

1. Azolylpropyl-oximino-Derivate der allgemeinen Formel

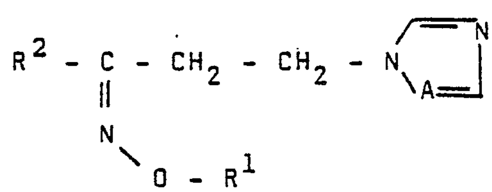

(I)

in welcher

A für Stickstoff oder die CH-Gruppe steht,

$R^1$ für Alkyl mit 1 bis 8 Kohlenstoffatomen, Alkenyl und Alkinyl mit jeweils 2 bis 6 Kohlenstoffatomen steht, sodann für Cyclopentyl, Cyclohexyl, Cyclohexylmethyl und Cyclohexenyl steht, sowie für gegebenenfalls einfach bis dreifach, gleichartig oder verschieden, durch Fluor, Chlor, Brom, Cyano, Nitro, Methyl, Ethyl, Methoxy, Methylendioxo, Phenyl, Phenoxy und/oder Trifluormethyl substituiertes Benzyl oder Phenyl steht, und

$R^2$ für substituiertes Phenyl steht, welches einfach oder zweifach, gleichartig oder verschieden durch Fluor, Chlor, Brom und Methyl, sowie durch Phenyl oder Phenoxy substituiert ist, wobei die beiden letztgenannten Zweitsubstituenten selbst noch gegebenenfalls durch Chlor, Brom oder Nitro substituiert sein können, sowie deren physiologisch verträglichen Säure-additions-Salze und Metallsalz-Komplexe.

2. Verfahren zur Herstellung von Azolylpropyl-oximino-Derivaten der allgemeinen Formel

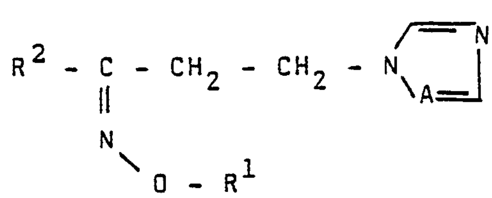

(I)

in welcher

A für Stickstoff oder die CH-Gruppe steht,

$R^1$ für Alkyl mit 1 bis 8 Kohlenstoffatomen, Alkenyl und Alkinyl mit jeweils 2 bis 6 Kohlenstoffatomen steht, sodann für Cyclopentyl, Cyclohexyl, Cyclohexylmethyl und Cyclohexenyl steht, sowie für gegebenenfalls einfach bis dreifach, gleichartig oder verschieden, durch Fluor, Chlor, Brom, Cyano, Nitro, Methyl, Ethyl, Methoxy, Methylendioxo, Phenyl, Phenoxy und/oder Trifluormethyl substituiertes Benzyl oder Phenyl steht, und

$R^2$ für substituiertes Phenyl steht, welches einfach oder zweifach, gleichartig oder verschieden durch Fluor, Chlor, Brom und Methyl, sowie durch Phenyl oder Phenoxy substituiert ist, wobei die beiden letztgenannten Zweitsubstituenten selbst noch gegebenenfalls durch Chlor, Brom oder Nitro substituiert sein können,

sowie von deren physiologisch verträglichen Säure-additions-Salzen und Metallsalz-Komplexen, dadurch gekennzeichnet, daß man

a) Azolyl-ketone der Formel

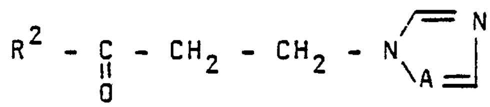

(II)

in welcher

A und $R^2$ die oben angegebene Bedeutung haben,

mit substituierten Hydroxylaminen der Formel

$$H_2N—O—R^1 \qquad (III)$$

in welcher

$R^1$ die oben angegebene Bedeutung hat,

in Gegenwart eines Verdünnungsmittels umsetzt, oder

b) Azolyl-oxime der Formel

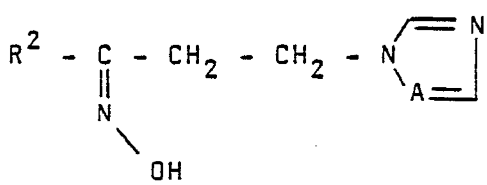

(IV)

in welcher

A und $R^2$ die oben angegebene Bedeutung haben,

mit Halogeniden der Formel

$$Hal—R^1 \qquad (V)$$

in welcher

$R^1$ die oben angegebene Bedeutung hat und

Hal für Chlor oder Brom steht,

gegebenenfalls in Gegenwart einer starken Base und in Gegenwart eines Verdünnungsmittels umsetzt, oder

c) Halogenpropyl-oximino-Derivate der Formel

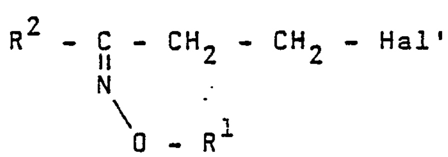

(VI)

in welcher

$R^1$ und $R^2$ die oben angegebene Bedeutung haben und

Hal′ für Chlor oder Brom steht,

mit 1,2,4-Triazol oder Imidazol in Gegenwart eines Säurebindemittels und in Gegenwart eines Verdünnungsmittels umsetzt,

und gegebenenfalls an die so erhaltenen Verbindungen der Formel (I) noch eine Säure oder ein Metallsalz addiert.

3. Fungizide und das Pflanzenwachstum regulierende Mittel, gekennzeichnet durch einen Gehalt an mindestens einem Azolylpropyl-oximino-Derivat der Formel (I) in Ansprüchen 1 und 2.

4. Verwendung von Azolylpropyl-oximino-Derivaten der Formel (I) in Ansprüchen 1 und 2 zur Bekämpfung von phytopathogenen Pilzen und zur Regulierung des Pflanzenwachstums.

**Claims**

1. Azolylpropyl-oximino derivatives of the general formula

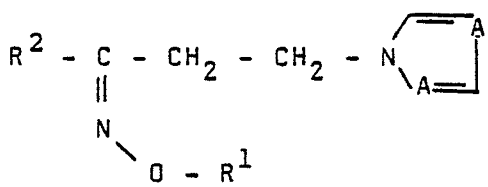

(I)

in which

A represents nitrogen or the CH group,

$R_1$ represents alkyl with 1 to 8 carbon atoms, alkenyl and alkinyl with in each case 2 to 6 carbon atoms, then cyclopentyl, cyclohexyl, cyclohexylmethyl and cyclohexenyl, and phenyl or benzyl optionally mono- to tri-substituted by identical or different substituents selected from fluorine, chlorine, bromine, cyano, nitro, methyl, ethyl, methoxy, methylenedioxo, phenyl, phenoxy and/or trifluoromethyl, and

$R^2$ represents substituted phenyl which is mono- or di-substituted by identical or different substituents selected from fluorine, chlorine, bromine and methyl, and phenyl or phenoxy, it being possible for the two last-mentioned secondary substituents themselves also to be optionally substituted by chlorine, bromine or nitro,

and physiologically tolerated acid addition salts and metal salt complexes thereof.

2. Process for the preparation of azolylpropyl-oximino derivatives of the general formula

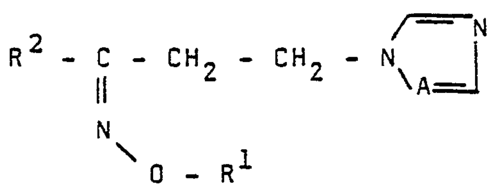

(I)

in which

A represents nitrogen or the CH group,

$R^1$ represents alkyl with 1 to 8 carbon atoms, alkenyl and alkinyl with in each case 2 to 6 carbon atoms, then cyclopentyl, cyclohexyl, cyclohexylmethyl and cyclohexenyl, and phenyl or benzyl optionally mono- to tri-substituted by identical or different substituents selected from fluorine, chlorine, bromine, cyano, nitro, methyl, ethyl, methoxy, methylenedioxo, phenyl, phenoxy and/or trifluoromethyl, and

$R^2$ represents substituted phenyl which is mono- or di-substituted by identical or different substituents selected from fluorine, chlorine, bromine and methyl, and phenyl or phenoxy, it being possible for the two last-mentioned secondary substituents themselves also to be substituted by chlorine, bromine or nitro, and physiologically tolerated acid addition salts and metal salt complexes thereof, characterised in that

a) azolyl-ketones of the formula

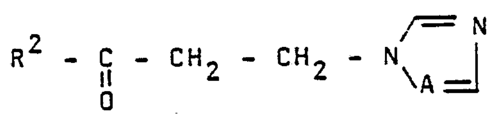

(II)

in which

A and $R^2$ have the abovementioned meaning, are reacted with substituted hydroxylamines of the formula

$$H_2N{-}O{-}R^1 \qquad (III)$$

in which

$R^1$ has the abovementioned meaning, in the presence of a diluent, or

b) azolyl-oximes of the formula

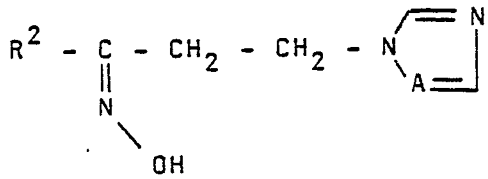

(IV)

in which

A and $R^2$ have the abovementioned meaning, are reacted with halides of the formula

$$Hal—R^1 \quad (V)$$

in which

$R^1$ has the abovementioned meaning and Hal represents chlorine or bromine, if appropriate in the presence of a strong base and in the presence of a diluent, or

c) halogenopropyl-oximino derivatives of the formula

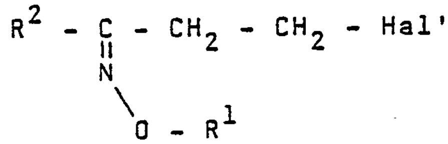

(VI)

in which

$R^1$ and $R^2$ have the abovementioned meaning and

Hal' represents chlorine or bromine,

are reacted with 1,2,4-triazole or imidazole in the presence of an acid-binding agent and in the presence of a diluent, and, if desired, an acid or a metal salt is then added on to the compounds of the formula (I) thus obtained.

3. Fungicides and agents regulating plant growth, characterised in that they contain at least one azolylpropyl-oximino derivative of the formula (I) in Claims 1 and 2.

4. Use of azolylpropyl-oximino derivatives of the formula (I) in Claims 1 and 2 for combating phytopathogenic fungi and for regulating plant growth.

**Revendications**

1. Dérivés azolylpropyl-oximinés de formule générale

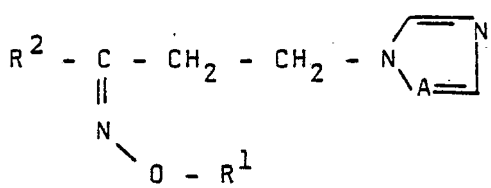

(I)

dans laquelle

A représente l'azote ou le groupe CH,

$R^1$ représente un groupe alkyle en $C_1$-$C_8$, alcényle et alcynyle contenant chacun 2 à 6 atomes de carbone, ou encore cyclopentyle, cyclohexyle, cyclohexylméthyle et cyclohexényle, ou également benzyle ou phényle portant éventuellement 1 à 3 substituants identiques ou différents choisis parmi le fluor, le chlore, le brome, les groupes cyano, nitro, méthyle, éthyle, méthoxy, méthylène-dioxo, phényle, phénoxy et/ou trifluorométhyle et,

$R^2$ représente un groupe phényle substitué, portant 1 ou 2 substituants identiques ou différents choisis parmi le fluor, le chlore, le brome et les groupes méthyle ou les groupes phényle ou phénoxy, ces deux derniers substituants pouvant eux-mêmes être éventuellement substitués par le chlore, le brome ou des groupes nitro, et leurs sels formés par addition avec des acides et complexes de sels métalliques tolérés par l'organisme.

2. Procédé de préparation des dérivés azolylpropyl-oximinés de formule générale

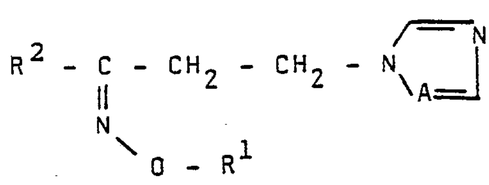

(I)

dans laquelle

A représente l'azote ou le groupe CH,

$R^1$ représente un groupe alkyle en $C_1$-$C_8$, alcényle et alcynyle contenant chacun 2 à 6 atomes de carbone, ou encore cyclopentyle, cyclohexyle, cyclohexylméthyle et cyclohexényle, ou également benzyle ou phényle portant éventuellement 1 à 3 substituants identiques ou différents choisis parmi le fluor, le chlore, le brome, les groupes cyano, nitro, méthyle, éthyle, méthoxy, méthylène-dioxo, phényle, phénoxy et/ou trifluorométhyle, et

$R^2$ représente un groupe phényle substitué, portant 1 à 2 substituants identiques ou différents choisis parmi le fluor, le chlore, le brome et les groupes méthyle ou les groupes phényle ou phénoxy, ces deux derniers substituants pouvant eux-mêmes être éventuellement substitués par le chlore, le brome ou des groupes nitro,

et de leurs sels formés par addition avec des acides et complexes de sels métalliques tolérés par l'organisme, caractérisé en ce que :

a) on fait réagir des azolyl-cétones de formule

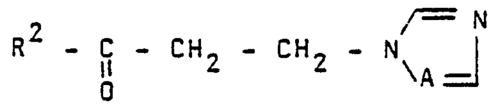

(II)

dans laquelle A et $R^2$ ont les significations indiquées ci-dessus, avec des hydroxylamines substituées de formule

$$H_2N—O—R^1 \qquad (III)$$

dans laquelle $R^1$ a les significations indiquées ci-dessus, en présence d'un diluant, ou bien

b) on fait réagir des azolyl-oximes de formule

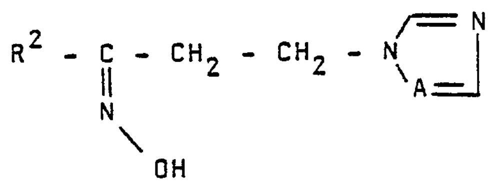

(IV)

dans laquelle A et $R^2$ ont les significations indiquées ci-dessus, avec des halogénures de formule

$$Hal—R^1 \qquad (V)$$

dans laquelle $R^1$ a les significations indiquées ci-dessus, et Hal représente le chlore ou le brome, éventuellement en présence d'une base forte et en présence d'un diluant, ou bien

c) on fait réagir des dérivés halogénopropyl-oximinés de formule

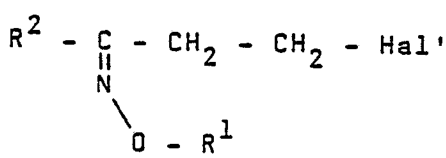

(VI)

dans laquelle $R^1$ et $R^2$ ont les significations indiquées ci-dessus, et Hal' représente le chlore ou le brome, avec le 1,2,4-triazole ou l'imidazole en présence d'un agent fixant les acides et en présence d'un diluant, et éventuellement, sur les composés de formule I ainsi obtenus, on fixe encore un acide ou un sel métallique par addition.

3. Produits fongicides et régulateurs de la croissance des végétaux, caractérisés en ce qu'ils contiennent au moins un dérivé azolylpropyl-oximiné de formule I des revendications 1 et 2.

4. Utilisation des dérivés azolylpropyl-oximinés de formule I des revendications 1 et 2, dans la lutte contre les mycètes phytopathogènes et la régulation de la croissance des végétaux.